# EUROPÄISCHE PATENTSCHRIFT

(11) **EP 3 682 152 B1**
(45) Veröffentlichungstag und Bekanntmachung des Hinweises auf die Patenterteilung: **12.08.2026**
(21) Anmeldenummer: 18772708.6
(22) Anmeldetag: 11.09.2018
(51) Int. Cl.: F16L 47/24, B29C 70/16, B29C 70/44, B29C 45/00, B29C 70/48

(54) **KUNSTSTOFF-COMPOSITE-BAUTEIL UMFASSENDE VORRICHTUNG**
DEVICE COMPRISING A PLASTIC-COMPOSITE COMPONENT
DISPOSITIF COMPRENANT UNE PIÈCE COMPOSITE EN MATIÈRE PLASTIQUE

(30) Priorität: 11.09.2017 CH 11272017
(43) Veröffentlichungstag der Anmeldung: 22.07.2020
(73) Patentinhaber: Kunststoffwerk AG Buchs, 9470 Buchs (CH)
(72) Erfinder: GSCHWEND, Oliver, 9470 Buchs (CH); RUDOLPH, Martin, 9470 Buchs (CH)
(74) Vertreter: Swisspat Riederer Hasler Patentanwälte AG
(86) Internationale Anmeldenummer: PCT/CH2018/050024
(87) Internationale Veröffentlichungsnummer: WO 2019/046977

(56) Entgegenhaltungen:
- EP-A1- 1 400 741
- DE-T2- 69 721 032
- US-B1- 6 450 547
- US-B1- 7 108 295
- BURKLE E ET AL: "VERBUNDBAUTEILE MIT MEHRPROZESSANLAGEN WIRTSCHAFTLICH HERSTELLEN", KUNSTSTOFFE, CARL HANSER VERLAG, MUNCHEN, DE, vol. 81, no. 3, 1 March 1991 (1991-03-01), pages 192 - 198, XP000287480, ISSN: 0023-5563

## Beschreibung

Die Erfindung betrifft eiine Vorrichtung nach dem Oberbegriff von Anspruch 1.

### Stand der Technik

Der heutige Wettbewerbsdruck zwingt die Firmen, bestehende Herstellungsverfahren und Produkte nach Einsparmöglichkeiten zu untersuchen. Wo immer möglich, werden zeit- und arbeitsintensive Arbeitsschritte bei der Herstellung von komplexen Metallbauteilen dadurch umgangen, dass Produkte vereinfacht werden, um diese dann automatisiert fertigen zu können. Auch gibt es eine Tendenz, teurere Metallbauteile durch günstigere Kunststoffeile zu ersetzen. Ein Problem dabei ist jedoch, dass die günstigeren Kunststoffteile sehr oft die Anforderungen bezüglich Stabilität nicht zu erfüllen vermögen. Aus diesem Grunde müssen die Kunststoffteile in der Regel grössere Wandstärken aufweisen als die entsprechenden Metallbauteile.

US 7,108,295 offenbart eine Verbundkupplung zur Verwendung beim Zusammenbau einer eingeschränkten Verbindung zwischen Rohren grossen Durchmessers, d.h. Durchmessern grösser als ca. 40 cm, mit äußeren komplementären Einspannnuten, die von den Rohrenden axial beabstandet sind. Die Verbundkupplung hat erste und zweite Rückhaltenuten im Inneren der Kupplung, die jeweils axial von dem Rohrenden beabstandet sind. Diese sind von aussen durch Löcher zugänglich, durch die ein Haltekeil eingeführt werden kann. Mittig im Innern der Kupplung ist ein interner Anschlag vorgesehen, an dem die zu verbindenden Rohre mit ihren Enden anliegen. Zwischen dem internen Anschlag und den Rückhaltenuten sind Nuten für die Aufnahme jeweils eines O-Rings vorgesehen. Die zylindrische Verbundkupplung weist eine Mehrzahl von konzentrisch angeordneten Schichten aus gewickelten Filamenten in einer duroplastischen Kunststoffmatrix auf.

EP-A-1 400 141 betrifft eine Kupplung zum zugfesten Zusammenfügen zweier Rohre aus Kunststoffmaterial. Die Rohre sind an ihren gegenüberliegenden Enden mit Flanschen versehen, und eine Hülse ist um jedes Rohr herum angeordnet. Die beiden Hülsen und die zwischen den Hülsen vorhandenen Flansche sind von einer weiteren äußeren Hülse umgeben. Im Außenumfang jeder ein Rohr umgebenden Hülse ist eine Nut gebildet, die gegenüber einer im Innenumfang der äußeren Hülse gebildeten Nut positioniert ist, wobei in jedem Paar einander gegenüberliegender Nuten ein Verriegelungselement vorhanden ist. Dieses verhindert, dass sich die Hülsen in Längsrichtung der Rohre zueinander bewegen. Die Kupplung ist weiter dadurch gekennzeichnet, dass die Flansche aneinander stoßen und jede Hülse locker um jedes Rohr herum angebracht werden kann und mit einem Ende gegen den Flansch des Rohres stößt. In der Nähe der Nuten sind Löcher vorhanden, über die Verriegelungsmittel in Form von Schnüren oder Bändern aus Kunststoffmaterial in die Nuten eingeführt werden können. Die Schnüre oder Bänder verhindern, dass sich die Hülsen in Längsrichtung der Rohre gegenüber der äußeren Hülse bewegen. Für die Hülsen wird ein glasfaserverstärkter Kunststoff verwendet.

Im Innenumfang der Hülse sind weitere kreisrunde Nuten gebildet, in denen O-Ringen vorgesehen sind. Die O-Ringe bilden im zusammengebauten Zustand der Kupplung eine Dichtung zwischen dem Innenumfang der äusseren Hülse und dem Außenumfang der Flansche. Da die Dichtungsringe an den Außenseiten der Kunststoffflansche angreifen, haben die Zugkräfte, die in axialer Richtung auf die Rohre ausgeübt werden können, keinen nachteiligen Einfluss auf die Dichtung. Es ist nicht angegeben, ob der Kunststoff mit Fasern verstärkt ist oder nicht.

Die DE 19902456 offenbart ein Verfahren und eine Anlage zur Herstellung von geschleuderten Glasfaserrohren mit mehreren Schichten aus geschnittenen Glasfasern. Geschnittene Glasfasern haben üblicherweise eine Länge zwischen 0,5 und 3mm.

### Aufgabe der Erfindung

Es ist deshalb ein Ziel der vorliegenden Erfindung, ein Bauteil zur Verfügung zu stellen, das Metallbauteile zu ersetzen vermag. Ein weiteres Ziel ist es, ein Bauteil zur Verfügung zu stellen, mit dem eine gute Dichtwirkung erzielt werden kann. Noch ein Ziel ist, ein Bauteil bereitzustellen, das als Gehäuse oder Bride verwendet werden kann und bevorzugt bei erhöhter Temperatur eingesetzt wird. Noch ein Ziel ist es, eine

### Beschreibung

Erfindungsgemäss werden die vorerwähnten Ziele durch die Merkmale von Anspruch 1 realisiert. Vorteilhafte Weiterbildungen sind in den Unteransprüchen definiert.

Die Erfindung betrifft ein hohles, insbesondere kreiszylindrisches Kunststoff-Composite-Bauteil zur Verwendung als vorzugsweise selbstdichtendes Gehäuse oder vorzugsweise selbstdichtende Bride oder Kupplung in Verbindung mit einem oder zwei vom Composite-Bauteil zu umschliessenden Bauteilen, insbesondere Metall-Bateilen. Das Kunststoff-Composite-Bauteil besitzt einen durch eine Wand gebildeten Mantel, an dessen Innenseite zwei Dichtungen in Längsrichtung des Bauteils gesehen im Abstand voneinander und vorzugsweise am Rand vorgesehen sind. Diese Dichtungen verlaufen mindestens abschnittsweise und vorzugsweise rundherum in Umfangsrichtung des Bauteils. Ausserdem sind im Kunststoff Endlosfasern eingebettet, die mehrheitlich im Wesentlichen quer zur Längsachse in Umfangsrichtung des Mantels verlaufend im Kunststoff angeordnet sind. Die in Umfangsrichtung verlaufenden Endlosfasern sollen dabei eine Faserlänge aufweisen, die mindestens ungefähr dem einfachen und vorzugsweise dem vielfachen, d.h. mehr als zehnfachen Umfang des Bauteils entspricht. Dies hat den Vorteil, dass das Kunststoff-Composite-Bauteil sich bei Erwärmung nur unwesentlich ausdehnt. Wird das vom Composite-Bauteil umschlossene Bauteil, insbesondere Metall-Bauteil, materialmässig so ausgewählt, dass es sich bei einem Temperaturanstieg stärker ausdehnt als das Composite-Bauteil, dann kommt es bei einer Erwärmung zu einem sich selbst verstärkenden Dichtungseffekt. Ein solches Kunststoff-Composite-Bauteil kann deshalb bestens schwerere Metallbauteile ersetzen, die ohne separate Dichtungen nicht auskommen.

Aus einer anderen Sichtweise ist das Kunststoff-Composite-Bauteil ein Rohr oder ein Ring mit einer Wand, die mindestens zwei Schichten oder Lagen aufweist, nämlich eine erste Schicht mit einem Fasergelege oder Fasergewebe, wobei zwischen zwei Enden des Fasergeleges oder Fasergewebes eine Stossverbindung definiert ist, und eine zweite Schicht, die mit der ersten Schicht vollflächig verbunden ist. Dabei kann die Stossverbindung zwischen zwei Kanten von verschiedenen Fasergelegen oder Fasergeweben im Falle eines nicht-runden Bauteils oder zwischen den Enden des gleichen Fasergeleges oder Fasergewebes im Falle eines runden Bauteils gebildet sein.

Das Fasergelege oder Fasergewebe ist dabei als vorgefertigtes Halbzeug eingesetzt.

Unter einem vorgefertigten Halbzeug soll im Rahmen der Erfindung verstanden werden, dass die Fasergelege oder Fasergewebe so zugeschnitten und/oder geformt sind, dass zwei Kanten aneinanderstossen oder sehr nah beieinander liegen, d.h. durch einen Spalt von maximal 1mm, vorzugsweise weniger als 0,5mm oder besonders bevorzugt noch weniger voneinander getrennt sind.

Vorteilhafterweise können die im Kunststoff eingebetteten Endlosfasern eine äussere Schicht der Wand bilden, an die eine weitere Kunststoffschicht in einer Spritzgiessvorrichtung angespritzt ist. Dabei ist die äussere Schicht mit den Endlosfaser maximal halb so dick, und vorzugsweise maximal ein Drittel und besonders bevorzugt ein Viertel so dick wie die gesamte Stärke der Wand.

Vorteilhaft liegen die Endlosfasern als Fasergewebe oder Fasergelege vor, wobei die Fasern mehrheitlich unidirektional im Wesentlichen in Umfangsrichtung ausgerichtet sind. Dies hat den Vorteil, dass das Kunststoff-Composite-Bauteil sehr formstabil ist und sich mit der Temperatur nur unwesentlich und auf jeden Fall weniger stark als Metall ausdehnt.

Grundsätzlich denkbar ist jedoch auch, dass das Halbzeug ein auf einem Trägermaterial angeordnetes Fasergelege oder Fasergewebe ist. Alternativ kann das eingesetzte Fasergelege auch durch eine Anzahl Schussfäden zusammengehalten sein derart, dass es sich zuschneiden und Händeln lässt.

Herstellungstechnisch wird das entsprechend zugeschnittene Faser-Halbzeug in eine Spritzgiessform eingelegt und dann umspritzt. Im Falle von in einer Kunststoffmatrix eingebetteten Faser Halbzeugen wird die zweite Schicht in einer Spritzgiessvorrichtung an die erste Schicht mit dem Halbzeug angespritzt.

Bevorzugt bilden das Fasergewebe oder Fasergelege eine äussere Lage der Wand. Herstellungstechnisch wird dazu vorzugsweise ein Halbzeug bestehend aus einem in einer Kunststoffmatrix eingebetteten Fasergewebe oder ein Fasergelege eingesetzt, das bereits die Gestalt des endgültigen Produkts hat. Dieses Halbzeug wird in eine Spritzgiessform eingelegt, sodass sich die einander gegenüberliegenden Ende auf Stoss berühren oder nur ein kleiner Spalt zwischen den Enden vorhanden ist, und dann eine weitere Kunststoffschicht vorzugsweise einseitig angespritzt. Denkbar ist jedoch auch, das Halbzeug mit Kunststoff beidseitig zu umspritzen.

Um die thermische Ausdehnung des Kunststoff-Composite-Bauteils auf ein Minimum zu beschränken, verlaufen vorzugsweise mehr als 60%, vorzugsweise mehr 75% und besonders bevorzugt mehr als 90% der Endlosfasern des Geleges oder des Gewebes in Umfangsrichtung des Bauteils. In einer bevorzugten Ausführungsform verlaufen im Wesentlichen alle Filamente in Umlaufrichtung, d.h. dass das Halbzeug vorzugsweise gewickelt ist. Damit können Kunststoff-Composite-Bauteile hergestellt werden, die einen kleineren thermischen Ausdehnungskoeffizienten aufweisen als ein vom Kunststoff-Composite-Bauteil zu umschliessendes Metall-Bauteil.

Vorteilhaft ist zur Herstellung des Kunststoff-Composite-Bauteils das Fasergelege oder Fasergewebe vorzugsweise als Halbzeug in einer Kunststoffmatrix eingebettet eingesetzt. Solchen eingebetteten Fasergelege oder -gewebe sind als sogenannte Fasertapes auf dem Markt erhältlich. Diese sind weniger als 1 mm dick und können beliebig zugeschnitten werden. An das in eine Spritzgiessform eingelegte Fasertape kann dann eine weitere Kunststoffschicht in einer Spritzgiessvorrichtung einseitig angespritzt werden, wobei die Kunststoffmatrix des Fasertapes und der anzuspritzende Kunststoff miteinander kompatibel sein müssen und bevorzugt zur gleichen Kunststoffgruppe gehören.

Zweckmässigerweise beträgt der gewichtsmässige Faseranteil in der eingesetzten Kunststoffmatrix zwischen 30% und 90%, vorzugsweise zwischen 40% und 80% und besonders bevorzugt zwischen 50% und 70%.

Vorteilhaft weist die Kunststoffmatrix mit dem darin eingebetteten Fasergelege oder Fasergewebe eine Stärke zwischen 0.1 und 1.5 mm, vorzugsweise zwischen 0.2 und 1.2 mm und besonders bevorzugt zwischen 0.4 und 1.0 mm auf. Vorzugsweise ist die Stärke der restlichen Wand ungefähr mindestens gleich gross, vorzugsweise mindest doppelt und besonders bevorzugt mindestens dreifach so gross wie die Kunststoffmatrix mit dem darin eingebetteten Fasergelege oder Fasergewebe.

Vorzugsweise sind als Endlosfasern Glas-, Aramid oder Kohlestofffasern eingesetzt, wobei Kohlestofffasern bevorzugt sind. Insbesondere Kohlestofffasern weisen einen sehr kleinen thermischen Ausdehnungskoeffizienten aus, was selbige für den Einsatz im Kunststoff-Composite-Bauteil prädestiniert.

Gemäss einer bevorzugten Ausführungsform sind die Dichtungen an der Innenseite des Bauteils angespritzt. Dies hat den Vorteil, dass diese unlösbar mit dem Kunststoff-Composite-Bauteil verbunden sind und nicht verrutschen können. Vorteilhaft sind die Dichtungen in einer Rille an der Innenwandung des Bauteils aufgenommen. Dadurch lässt sich eine gute Verbindung zwischen Innenwand und Dichtung erreichen. Zweckmässigerweise sind die Dichtungen aus einem Elastomeren hergestellt. Als Elastomere können Copolyamide,z.B. PEBAX, thermoplastische Copolyester, Elastomere auf Urethan- oder Styrolbasis eingesetzt sein.

Vorteilhaft ist als Kunststoff ein solcher aus der Gruppe der Polycarbonate, Polyamide, Polyphenylensulfide (PPS), Polysulfone, Polyethylene, Polybutylenterephthalaet oder Polyetheretherketone eingesetzt, wobei Polyamid 6 oder Polyamid 66 bevorzugt ist.

Gegenstand der vorliegenden Beschreibung ist eine Vorrichtung resp. ein Bauteil mit einem oben beschriebenen Kunststoff-Composite-Bauteil und einem oder zwei Innenteilen aus Metall, wobei das Kunststoff-Composite-Bauteil auf das oder die Innenteile aufsetzbar ist und dadurch entweder ein zwischen den beiden Innenteilen vorhandener Spalt oder ein zwischen dem Innenteil und dem Aussenteil vorhandener Zwischenraum, insbesondere Ringraum, durch die voneinander beabstandeten Dichtungen des Kunststoff-Composite-Bauteils abgedichtet ist, und das Innenteil oder der Zwischenraum der Durchleitung eines Wärmetransfermediums dient. Diese Vorrichtung kann ein Teil einer Maschine oder einer anderen Vorrichtung sein, mit dem die Maschine oder Vorrichtung gekühlt wird.

Vorteilhaft ist die Vorrichtung ein aus dem einen Innenteil und dem Kunststoff-Composite-Bauteil gebildetes Bauteil, insbesondere Maschinenteil, wobei das Innenteil und das Kunststoff-Composite-Bauteil einen Zwischenraum, insbesondere einen Ringraum, für die Durchleitung eines Wärmetransfermediums definieren. Dadurch ist zwischen dem Innenteil und dem Kunststoff-Composite-Bauteil ein fluiddichter Zwischenraum für die Durchleitung eines Wärmetransfermediums gebildet ist.

In einer vorteilhaften Ausführungsform ist der Zwischenraum durch zwei Öffnungen zugänglich und durch einen Steg resp. Wand unterbrochen derart, dass ein durch die eine Öffnung in den Zwischenraum geleitetes Wärmetransfermedium bis zur zweiten Öffnngen möglichst den ganzen Zwischenraum durchströmen kann.

Vorzugsweise ist der Zwischenraum durch zwei Öffnungen und durch einen Steg resp. Wand unterbrochen derart, dass ein durch die eine Öffnung in den Zwischenraum geleitetes Wärmetransfermedium bis zur zweiten Öffnngen möglichst den ganzen Zwischenraum durchströmen kann.

Vorteilhaft ist das Kunststoff-Composite-Bauteil so ausgelegt, dass es einen kleineren thermischen Ausdehnungskoeffizienten aufweist als das vom Kunststoff-Composite-Bauteil umschlossene Metall-Bauteil. Dadurch kann ein sich selbstverstärkender Dichteffekt bewirkt werden, wenn sich die beiden Komponenten mit unterschiedlichen thermischen Ausdehnungskoeffizienten ausdehnen.

Ausführungsbeispiele der Erfindung werden nun unter Bezugnahme auf die nachfolgenden Figuren näher im Detail beschrieben. Es zeigt:
- Figur 1:: Eine perspektivische Ansicht eines erfindungsgemässen Kunststoff-Composite-Bauteils in Gestalt eines Rings;
- Figur 2:: Das Bauteil von Fig. 1 zusammen mit einem vom Bauteil umfassten Metallrohr im Querschnitt;
- Figur 3:: Einen Längsschnitt durch die Anordnung von Fig. 2 entlang der Linie A-A;
- Figur 4:: Ein Detailvergrösserung der Fig. 3.,
- Figur 5:: Eine Seitenansicht des Bauteils von Fig. 1; und
- Figur 6:: Einen Schnitt durch die Ringwand in stark vergrössertem Massstab.

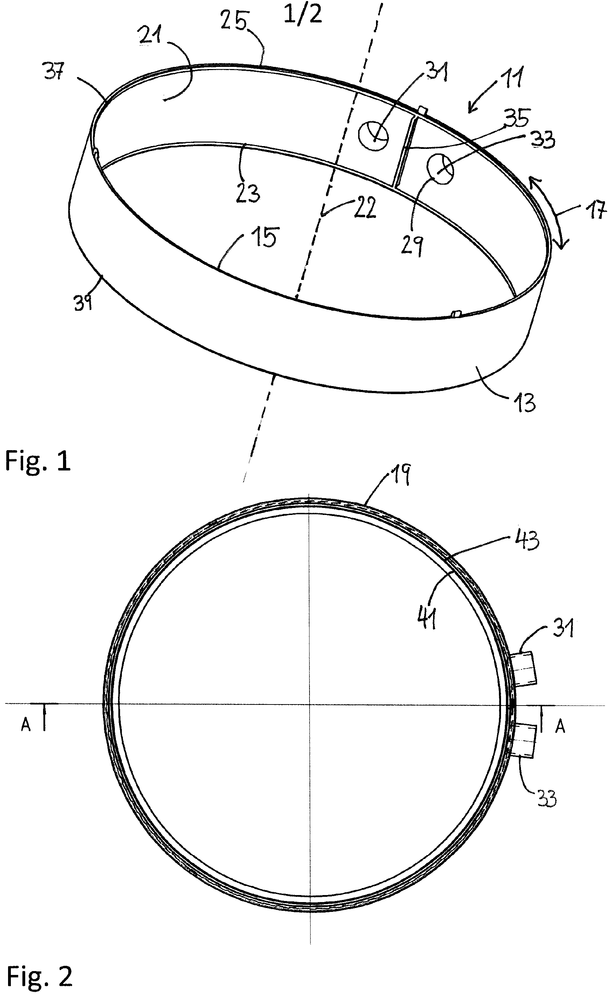

Die Figuren 1, 5 und 6 zeigen ein erfindungsgemässes Kunststoff-Composite-Bauteil 11 in Gestalt eines kreiszylindrischen Mantels 13, der als Gehäuse einsetzbar ist. Der Mantel 13 ist durch eine Wand 15 gebildet, in welcher in Umfangsrichtung (Pfeil 17) verlaufende Endlosfasern 19 eingebettet sind. An der Innenseite 21 des Composite-Bauteils 11 sind in Längsrichtung 22 des Bauteils gesehen im Abstand voneinander zwei Dichtungen 23, 25 vorgesehen, welche aus der Manteloberfläche herausragen. Vorliegend sind die Dichtungen 23,25 an den gegenüberliegenden Rändern des zylindrischen Bauteils vorgesehen. Die Dichtungen 23, 25 bestehen vorzugsweise aus einem Elastomeren und sind folglich elastisch deformierbar. Im Mantel 13 sind zwei Öffnungen 27,29 vorgesehen, von denen die eine als Einlass und die andere als Auslass für ein Wärmetransfermedium dient. An die Öffnungen 27,29 sind Stutzen 31,33 angeformt, an welche in den Figuren nicht gezeigte Schläuche anschliessbar sind. Zwischen den beiden Öffnungen 27,29 ist an der Innenseite 21 des Mantels 13 ein radial nach innen ragender Steg 35 vorgesehen. Der Steg 35 erstreckt sich zwischen den einander gegenüberliegenden Rändern 37,39 des Mantels 13 und dient dazu, in Verbindung mit einem Innenteil, z.B. einem Rohr oder Innengehäuse 41, einen zwischen dem Mantel 13 und dem Innengehäuse 41 vorhandenen Ringraum 43 zu unterteilen (Figuren 2 bis 4). Damit wird ein durch die Öffnung 27 einströmendes, gasförmies oder flüssiges Wärmetransfermedium gezwungen, im Wesentlichen den gesamten Ringraum 43 bis zur Auslassöffnung 29 zu durchströmen. Selbstverständlich könnte der Steg 35 anstatt an der Innenseite 21 des Mantels 13 auch am Innengeäuse 41 vorgesehen sein.

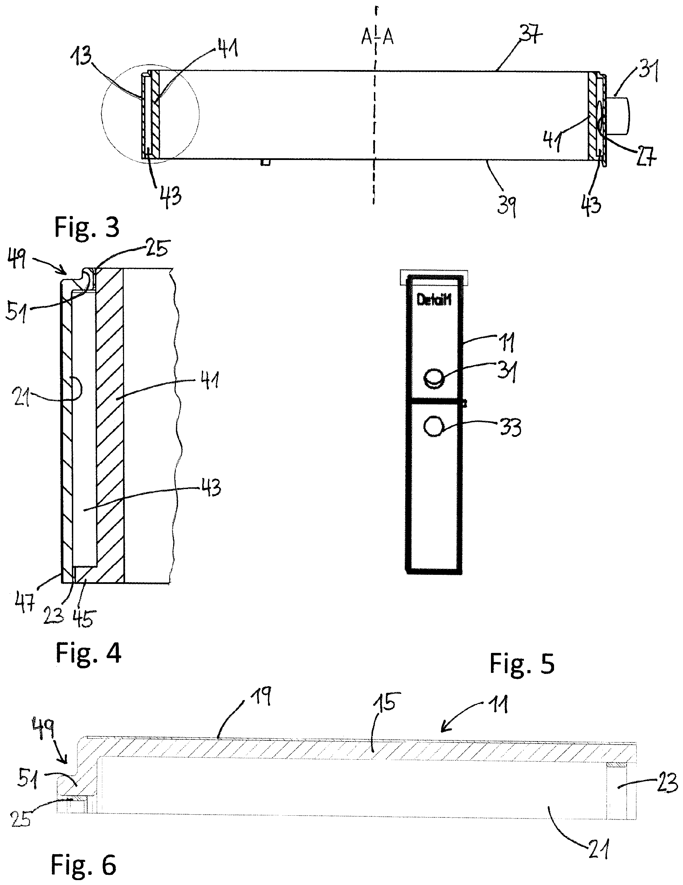

In Fig. 4 ist ein Schnitt durch das auf das Innengehäuse 41 aufgesetzte Kunststoff-Composite-Bauteil 11 näher im Detail gezeigt. Es ist erkennbar, dass das Innengehäuse 41 auf der einen Seite einen radial nach aussen abstehenden Ringsteg 45 aufweist, auf den ein Randabschnitt 47 des Kunststoff-Composite-Bauteils 11 aufgesetzt ist. An der gegenüberliegenden Seite besitzt das Kunststoff-Composite-Bauteil 11 einen zweiten Randabschnitt 49, der durch einen nach innen ragenden L-förmigen Flansch 51 gebildet ist. An der Innenseite der Randabschnitte 47,49 sind die Dichtungen 23, 25 vorgesehen, die den Ringraum 43 nach aussen abdichten.

In Figur 6 ist der Aufbau der Wand 15 näher im Detail gezeigt. Es ist erkennbar, dass die Endlosfasern 19 eine einzelne äussere Lage bilden, die maximal 30%, vorzugsweise maximal 20% und besonders bevorzugt maximal 15% der Gesamtwandstärke ausmacht. An den gegenüberliegenden Enden des auf beiden Seiten offenen Bauteils sind die Dichtungen 23,25 angespritzt.

Von Bedeutung für die Erfindung ist, dass die thermische Ausdehnung des Kunststoff-Composite-Bauteils 11 kleiner als diejenige des Innengehäuses 41 ist. Dies hat den Vorteil, dass bei der Erwärmung der beiden Teile die Dichtwirkung noch verstärkt wird.

Das Kunststoff-Composite-Bauteil 11 wird wie folgt hergestellt: Zuerst wird vorzugsweise eine Endlosfaser auf einen Grundkörper, der die Gestalt des herzustellenden Kunststoff-Composite-Bauteils aufweist, aufgewickelt und dann die Form mit einem flüssigen Kunststoff fixiert. Die Fixierung der Form kann durch Eintauchen des Grundkörpers mit der aufgewickelten Endlosfaser in einen flüssigen Kunststoff, Besprühen oder Umspritzen erfolgen. Denkbar ist auch, die Endlosfaser zuerst mit Kunststoff zu beschichten und dann die fertige Form noch einer Temperaturbehandlung zu unterziehen, in der der Kunststoff nochmals aufgeschmolzen wird. Danach wird das so erzeugte Halbzeug in eine Spritzgiessform eingebracht und mit einer Kunststoffschicht umspritzt oder vorzugsweise eine Kunststoffschicht einseitig angespritzt. Alternativ kann auch ein auf dem Markt erhältliches Fasertape eingesetzt werden, das ein in einer Kunststoffmatrix eingebettetes Fasergelege oder Fasergewebe aufweist. Das Fasertape wird so zugeschnitten, dass dessen Länge dem Umfang und dessen Breite der Breite des Bauteils entspricht. Dieses Fasertape wird in eine Spritzgiessform eingelegt und dann ein kompatibler Kunststoff angespritzt.

Die Erfindung betrifft ein hohles Kunststoff-Composite-Bauteil zur Verwendung als selbstdichtendes Gehäuse oder selbstdichtende Bride in Verbindung mit einem oder zwei vom Composite-Bauteil zu umschliessenden Metall-Bauteilen. Das Bauteil besteht aus einem durch eine Wand gebildeten Mantel, wobei an der Innenseite des Mantels zwei Dichtungen vorgesehen sind. Die Dichtungen sind in Längsrichtung des Bauteils gesehen im Abstand voneinander angeordnet und verlaufen in Umfangsrichtung. Im Kunststoff sind Endlosfasern eingebettet, die mehrheitlich im Wesentlichen quer zur Längsachse in Umfangsrichtung des Mantels verlaufen. Das Kunststoff-Composite-Bauteil zeichnet sich durch einen sehr kleinen thermischen Ausdehnungskoeffizienten aus.

### Legende

- 11: Kunststoff-Composite-Bauteil
- 13: Mantels
- 15: Wand
- 17: Pfeil
- 19: Endlosfasern
- 21: Innenseite
- 23, 25: Dichtungen
- 27,29: Öffnungen
- 31,33: Stutzen
- 35: Steg
- 37, 39: Ränder
- 41: Innengehäuse
- 43: Ringraum
- 47,49: Randabschnitte des Kunststoff-Composite-Bauteils
- 51: L-förmiger Flansch

## Patentansprüche

1. Vorrichtung mit einem hohlen, insbesondere kreiszylindrischen Kunststoff-Composite-Bauteil und einem oder zwei Innenteilen (41) aus Metall, wobei das Kunststoff-Composite-Bauteil
- einen durch eine Wand (15) gebildeten Mantel (13),
- in Längsrichtung des Bauteils gesehen zwei im Abstand voneinander an der Innenseite (21) des Mantels (15) vorgesehene und in Umfangsrichtung verlaufende Dichtungen (23,25), und
- im Kunststoff eingebettete Endlosfasern (19), die mehrheitlich im Wesentlichen quer zur Längsachse (22) in Umfangsrichtung des Mantels (13) verlaufend im Kunststoff angeordnet sind und eine äussere Schicht der Wand (15) bilden, an die eine weitere Kunststoffschicht angespritzt ist,
umfasst,
**dadurch gekennzeichnet, dass** das Kunststoff-Composite-Bauteil auf das oder die Innenteile (41) aufsetzbar ist und dadurch entweder ein zwischen den beiden Innenteilen vorhandener Spalt oder ein zwischen dem Innenteil und dem Aussenteil vorhandener Zwischenraum (43) durch die voneinander beabstandeten Dichtungen (23,25) des Kunststoff-Composite-Bauteils abgedichtet ist, und das Innenteil (41) oder der Zwischenraum (43) der Durchleitung eines Wärmetransfermediums dient.

2. Vorrichtung nach Anspruch 1, **dadurch gekennzeichnet, dass** die Vorrichtung ein aus einen Innenteil (41) und dem Kunststoff-Composite-Bauteil (11) gebildetes Bauteil, insbesondere Maschinenteil, ist, wobei zwischen dem Innenteil (41) und dem Kunststoff-Composite-Bauteil (11) ein mindestens fluiddichter Zwischenraum (43) für die Durchleitung eines Wärmetransfermediums gebildet ist.

3. Vorrichtung nach Anspruch 1 oder 2, **dadurch gekennzeichnet, dass** der Zwischenraum durch zwei Öffnungen zugänglich ist und durch einen Steg resp. Wand unterbrochen ist derart, dass ein durch die eine Öffnung in den Zwischenraum geleitetes Wärmetransfermedium bis zur zweiten Öffnngen möglichst den ganzen Zwischenraum durchströmen kann.

4. Vorrichtung nach einem der Ansprüche 1 bis 3, **dadurch gekennzeichnet, dass** das Kunststoff-Composite-Bauteil und das Innenteil (41) so ausgelegt sind, dass das Kunststoff-Composite-Bauteil einen kleineren thermischen Ausdehnungskoeffizienten aufweist als das vom Kunststoff-Composite-Bauteil umschlossene Metall-Bauteil (41).

5. Vorrichtung nach einem der Ansprüche 1 bis 4, **dadurch gekennzeichnet, dass** die Schicht mit den Endlosfasern entweder als Fasergewebe oder Fasergelege eingesetzt ist.

6. Vorrichtung nach einem der Ansprüche 1 bis 5, **dadurch gekennzeichnet, dass** die Schicht mit den Endlosfasern des Kunststoff-Composite-Bauteils als gewickeltes Halbzeug eingesetzt ist.

7. Vorrichtung nach einem der Ansprüche 1 bis 6, **dadurch gekennzeichnet, dass** im Kunststoff-Composite-Bauteil mehr als als 60%, vorzugsweise mehr 75% und besonders bevorzugt mehr als 90% der Endlosfasern in Umfangsrichtung des Bauteils verlaufen.

8. Vorrichtung nach einem der Ansprüche 1 bis 7, **dadurch gekennzeichnet, dass** zur Herstellung des Kunststoff-Composite-Bauteils das Fasergelege oder Fasergewebe als Halbzeug in einer Kunststoffmatrix eingebettet eingesetzt ist, an die eine weitere Kunststoffschicht in einer Spritzgiessvorrichtung angespritzt wird.

9. Vorrichtung nach einem der Ansprüche 6 bis 8, **dadurch gekennzeichnet, dass** der gewichtsmässige Faseranteil in der eingesetzten Kunststoffmatrix zwischen 30% und 90%, vorzugsweise zwischen 40% und 80% und besonders bevorzugt zwischen 50% und 70% beträgt.

10. Vorrichtung nach einem der Ansprüche 1 bis 9, **dadurch gekennzeichnet, dass** als Fasern Glas-, Aramid oder Kohlestofffasern, wobei Kohlestofffasern bevorzugt sind, eingesetzt sind.

11. Vorrichtung nach einem der Ansprüche 1 bis 10, **dadurch gekennzeichnet, dass** die Kunststoffmatrix im Kunststoff-Composite-Bauteil mit dem darin eingebetteten Fasergelege oder Fasergewebe eine Stärke zwischen 0.1 und 1.5 mm, vorzugsweise zwischen 0.2 und 1.2 mm und besonders bevorzugt zwischen 0.4 und 1.0 mm aufweist.

12. Vorrichtung nach einem der Ansprüche 1 bis 11, **dadurch gekennzeichnet, dass** die Dichtungen (23,25) im Kunststoff-Composite-Bauteil aus einem elastomeren Material an der Innenseite (21) des Bauteils angespritzt und vorzugsweise in einer Rille an der Innenseite (21) der Wand (15) aufgenommen sind.

13. Vorrichtung nach einem der Ansprüche 1 bis 12, **dadurch gekennzeichnet, dass** ein Kunststoff aus der Gruppe entweder der Polycarbonate, Polyamide, Polyphenylensulfide (PPS), Polysulfone, Polyethylene, Polybutylenterephthalate, Polyetheretherketone, Polyamid 6 oder Polyamid 66 eingesetzt ist.

## Claims

1. A device having a hollow, in particular circular cylindrical, plastic composite component and one or two inner parts (41) made from metal, wherein the plastic composite component comprises
- a casing (13) formed by a wall (15),
- two seals (23, 25) which are provided at a distance from one another on the inside (21) of the casing (15) and run in the circumferential direction, as viewed in the longitudinal direction of the component, and
- continuous fibres (19) which are embedded in plastic and which are arranged for the most part substantially transversely to the longitudinal axis (22) running in the circumferential direction of the casing (13) in the plastic, and forming an outer layer of the wall (15), onto which a further plastic layer is injection-moulded,
**characterized in that** the plastic composite component can be placed onto the inner part or the inner parts (41) and as a result, either a gap that is present between the two inner parts or an intermediate space (43) that is present between the inner part and the outer part is sealed by the mutually spaced seals (23, 25) of the plastic composite component, and the inner part (41) or the intermediate space (43) is used for channelling of a heat transfer medium.

2. The device according to Claim 1, **characterized in that** the device is a component formed from an inner part (41) and the plastic composite component (11), in particular a machine part, wherein an at least fluid-tight intermediate space (43) for the channelling of a heat transfer medium is formed between the inner part (41) and the plastic composite component (11).

3. The device according to Claim 1 or 2, **characterized in that** the intermediate space is accessible by means of two openings and is interrupted by a web or wall such that a heat transfer medium that is conducted through an opening into the intermediate space can flow to the greatest extent possible through the entire intermediate space as far as the second opening.

4. The device according to any one of Claims 1 to 3, **characterized in that** the plastic composite component and the inner part (41) are configured such that the plastic composite component has a smaller thermal coefficient of expansion than the metal component (41) surrounded by the plastic composite component.

5. The device according to any one of Claims 1 to 4, **characterized in that** the layer with the continuous fibres is used either as a fibre woven fabric or fibre fabric.

6. The device according to any one of Claims 1 to 5, **characterized in that** the layer with the continuous fibres of the plastic composite component is used as a wound semi-finished product.

7. The device according to any one of Claims 1 to 6, **characterized in that** in the plastic composite component, more than 60%, preferably more than 75% and particularly preferably more than 90% of the continuous fibres run in the circumferential direction of the component.

8. The device according to any one of Claims 1 to 7, **characterized in that** to produce the plastic composite component, the fibre fabric or fibre woven fabric is used as semi-finished product, embedded in a plastic matrix, onto which plastic matrix a further plastic layer is injection-moulded in an injection moulding device.

9. The device according to any one of Claims 6 to 8, **characterized in that** the fibre proportion in terms of weight in the plastic matrix used is between 30% and 90%, preferably between 40% and 80% and particularly preferably between 50% and 70%.

10. The device according to any one of Claims 1 to 9, **characterized in that** glass, aramid or carbon fibres are used as fibres, wherein carbon fibres are preferred.

11. The device according to any one of Claims 1 to 10, **characterized in that** the plastic matrix in the plastic composite component with the fibre fabric or fibre woven fabric embedded therein has a thickness between 0.1 and 1.5 mm, preferably between 0.2 and 1.2 mm and particularly preferably between 0.4 and 1.0 mm.

12. The device according to any one of Claims 1 to 11, **characterized in that** the seals (23, 25) in the plastic composite component are injection moulded from an elastomeric material on the inner side (21) of the component and preferably accommodated in a groove on the inner side (21) of the wall (15).

13. The device according to any one of Claims 1 to 12, **characterized in that** a plastic from the group either of the polycarbonates, polyamides, polyphenyl sulphides (PPS), polysulphones, polyethylenes, polybutylene terephthalates, polyether ether ketone, polyamide 6 or polyamide 66 is used.

## Revendications

1. Dispositif, doté d'un élément composite en matière plastique creux, notamment de forme cylindrique circulaire et d'une ou deux pièces internes (41) en métal, l'élément composite en matière plastique comprenant
- une enveloppe (13) constituée par une paroi (15),
- deux joints (23, 25), prévus sur la face intérieure (21) de l'enveloppe (15), avec un écart l'un de l'autre, considérés dans la direction longitudinale de l'élément et s'étendant dans la direction circonférentielle et
- des fibres continues (19) incorporées dans la matière plastique, qui en majorité, sont placées dans la matière plastique en s'étendant sensiblement à la transversale de l'axe longitudinal (22) dans la direction circonférentielle de l'enveloppe (13) et constituent une couche extérieure de la paroi (15), sur laquelle est surmoulée une couche supplémentaire de matière plastique,
**caractérisé en ce que** l'élément composite en matière plastique peut se monter sur la ou les pièces internes (41) et que de la sorte l'étanchéité soit d'une fente présente entre les deux pièces internes ou d'un espace intermédiaire (43) présent entre la pièce interne et la pièce externe soit assurée par les joints (23, 25) espacés l'un de l'autre de l'élément composite en matière plastique et **en ce que** la pièce interne (41) ou l'espace intermédiaire (43) est destiné(e) au passage d'un agent caloporteur.

2. Dispositif selon la revendication 1, **caractérisé en ce que** le dispositif est un élément constitué d'une pièce interne (41) et de l'élément composite en matière plastique (11), notamment un élément de machine, entre la pièce interne (41) et l'élément composite en matière plastique (11) étant constitué un espace intermédiaire (43) étanche au moins aux fluides, pour le passage d'un agent caloporteur.

3. Dispositif selon la revendication 1 ou 2, **caractérisé en ce que** l'espace intermédiaire est accessible à travers deux orifices et interrompu par un listel ou une paroi, de telle sorte qu'un agent caloporteur dirigé dans l'espace intermédiaire à travers l'un orifice puisse traverser jusqu'au deuxième orifices si possible la totalité de l'espace intermédiaire.

4. Dispositif selon l'une quelconque des revendications 1 à 3, **caractérisé en ce que** l'élément composite en matière plastique et la pièce interne (41) sont conçus de telle sorte que l'élément composite en matière plastique présente un coefficient de dilatation thermique inférieur à celui de la pièce (41) métallique entourée par l'élément composite en matière plastique.

5. Dispositif selon l'une quelconque des revendications 1 à 4, **caractérisé en ce que** la couche comportant les fibres continues est mise en œuvre soit sous la forme d'un tissu de fibres ou d'une nappe de fibres.

6. Dispositif selon l'une quelconque des revendications 1 à 5, **caractérisé en ce que** la couche comportant les fibres continues de l'élément composite en matière plastique est mise en œuvre sous la forme d'un produit semi-fini enroulé.

7. Dispositif selon l'une quelconque des revendications 1 à 6, **caractérisé en ce que** dans l'élément composite en matière plastique, plus de 60 %, de préférence plus de 75 % et de manière particulièrement préférentielle, plus de 90 % des fibres continues s'étendent dans la direction circonférentielle de l'élément.

8. Dispositif selon l'une quelconque des revendications 1 à 7, **caractérisé en ce que** pour la fabrication de l'élément composite en matière plastique, l'on met en œuvre la nappe de fibres ou le tissu de fibres sous la forme d'un produit semi-fini incorporé dans une matrice en matière plastique, sur laquelle l'on surmoule une couche supplémentaire de matière plastique dans un dispositif de moulage par injection.

9. Dispositif selon l'une quelconque des revendications 6 à 8, **caractérisé en ce que** la proportion en poids des fibres dans la matrice en matière plastique mise en œuvre est comprise entre 30 % et 90 %, de préférence entre 40 % et 80 % et de manière particulièrement préférentielle, entre 50 % et 70 %.

10. Dispositif selon l'une quelconque des revendications 1 à 9, **caractérisé en ce que** sont mises en œuvre en tant que fibres des fibres de verre, d'aramide ou de carbone, les fibres de carbone étant préférentielles.

11. Dispositif selon l'une quelconque des revendications 1 à 10, **caractérisé en ce que** la matrice en matière plastique dans l'élément composite en matière plastique présente avec la nappe de fibres ou le tissu de fibre incorporé(e) en elle une épaisseur comprise entre 0.1 et 1.5 mm, de préférence entre 0.2 et 1.2 mm et de manière particulièrement préférentielle, entre 0.4 et 1.0 mm.

12. Dispositif selon l'une quelconque des revendications 1 à 11, **caractérisé en ce que** les joints (23, 25) dans l'élément composite en matière plastique sont surmoulés en une matière élastomère sur la face intérieure (21) de l'élément et sont réceptionnés de préférence dans une gorge sur la face intérieure (21) de la paroi (15).

13. Dispositif selon l'une quelconque des revendications 1 à 12, **caractérisé en ce que** l'on met en œuvre une matière plastique du groupe, soit des polycarbonates, des polyamides, des poly (sulfures de phénylène) (PPS), des polysulfones, des polyéthylènes, des poly (téréphtalates de butylène), des poly(éther-éther-cétones), du polyamide 6 ou du polyamide 66.
